Europäisches Patentamt

European Patent Office

Office européen des brevets

(19)

(11) Veröffentlichungsnummer: **0 211 169**
B1

(12) **EUROPÄISCHE PATENTSCHRIFT**

(45) Veröffentlichungstag der Patentschrift:
18.10.89

(51) Int. Cl.⁴: **A 61 F 2/34**

(21) Anmeldenummer: **86107088.6**

(22) Anmeldetag: **24.05.86**

(54) Randlose Hüftgelenkspfanne.

(30) Priorität: **15.07.85 CH 3065/85**

(43) Veröffentlichungstag der Anmeldung:
**25.02.87 Patentblatt 87/9**

(45) Bekanntmachung des Hinweises auf die Patenterteilung:
**18.10.89 Patentblatt 89/42**

(84) Benannte Vertragsstaaten:
**AT DE FR GB IT**

(56) Entgegenhaltungen:
**DE-A- 2 318 459
DE-A- 3 228 113
DE-A- 3 406 357
FR-A- 2 478 462
US-A- 3 740 769**

(73) Patentinhaber: **GEBRÜDER SULZER
AKTIENGESELLSCHAFT, Zürcherstrasse 9,
CH-8401 Winterthur (CH)**

(72) Erfinder: **Griss, Peter, Prof. Dr.-med. Orthopädische
Klinik, Klinikum der Philipps-Universität,
D-3550 Marburg (DE)**

(74) Vertreter: **Dipl.-Ing. H. Marsch Dipl.-Ing. K. Sparing
Dipl.-Phys.Dr. W.H. Röhl Patentanwälte,
Rethelstrasse 123, D-4000 Düsseldorf 1 (DE)**

## Beschreibung

Die Erfindung betrifft eine randlose Hüftgelenkspfanne mit einem in die von der Symmetrieachse der Pfanne abweichende Einsetzrichtung weisenden, aus der äusseren Oberfläche hervorstehenden Zapfen.

Bei zementfreier Verankerung von Implantaten ist die sogenannte Primärfixation eines der wichtigsten Probleme. Darunter versteht man bekanntlich die Verankerung des Implantates unmittelbar nach dem Einsetzen bis zu einer endgültigen Fixierung durch an- oder einwachsendes Gewebe. Für die Primärfixation von Hüftgelenkspfannen wird dabei häufig ein Klemmsitz angewandt, bei dem die Pfanne in den Beckenknochen, beispielsweise mit Hilfe von einem oder mehreren aus ihrer Aussenfläche hervorragenden Zapfen und/oder mit Hilfe von Teilbereichen ihrer Aussenfläche, die vorzugsweise nahe dem äquatorialen Rand liegen, eingeklemmt werden (DE-A-3 341 724 und DE-A-3 341 723).

Es hat sich nun gezeigt, dass — besonders bei Konstruktionen, bei denen die Klemmung mindestens teilweise unmittelbar über dem Pfannenkörper erfolgt — Deformationen der eigentlichen Pfannenschale auftreten, die zu einem Einklemmen des Gelenkkopfes der zugehörigen Femurkopfprothese führen können.

Aufgabe der Erfindung ist es, eine Hüftgelenkspfanne für eine zementfreie Verankerung zu schaffen, bei der Verformungen der eigentlichen Pfannenschale möglichst vermieden werden, obwohl die Primärfixation über einen Klemmsitz erfolgt. Diese Aufgabe wird mit der Erfindung dadurch gelöst, dass aus dem Umfang des Pfannenkörpers in der Äquatorebene zwei Verankerungslaschen mit Bohrungen hervorragen, deren Achsen parallel zur Symmetrieachse des Pfannenkörpers verlaufen, und dass ferner die periphere Aussenform der Laschen ebenfalls mindestens nahezu parallel zur Symmetrieachse des Pfannenkörpers verläuft, so dass im eingebauten Zustand zwischen dem Zapfen und den Laschen ein Klemmsitz zur Primärfixierung der Pfanne entsteht; es ist jedoch auch möglich, dass die periphere Aussenform der Laschen mit der Symmetrieachse des Pfannenkörpers eine zum Pfannenscheitel offenen spitzen Winkel bildet.

Die bei einem Klemmsitz auftretenden Deformationen am Pfannenkörper treten bei der neuen Pfanne weitgehend in und an den Laschen auf, so dass vor allem die Randbereiche des Pfannenkörpers, wenn überhaupt, nur geringfügig verzerrt werden. Die Primärfixation durch den Klemmsitz wird durch Schrauben, mit denen die Laschen im Knochen verschraubt werden, zusätzlich verstärkt, wobei diese Schrauben gleichzeitig die Laschen fixieren und einer Deformation entgegenwirken.

Für eine Optimierung der Schraubverbindung mit dem Knochen hat es sich als zweckmässig herausgestellt, wenn die Bohrungen zur Äquatorebene hin zu Hohlkugelflächen und etwa von der Mitte der Laschenhöhe ab gegen den Knochen hin zu einem Konus erweitert sind, da dadurch in gewissem Umfang die Einschraubrichtung für die Knochenschrauben verändert werden kann.

Für die Wirksamkeit des Klemmsitzes ist es vorteilhaft, wenn die Laschenhöhe von dem Winkel $\alpha$ abhängt, den die Zapfenachse mit der Symmetrieachse des Pfannenkörpers bildet, wobei die Laschenhöhe bei grossen Winkeln $\alpha$ relativ klein ist und umgekehrt; denn je grösser der Winkel $\alpha$ ist, der ja die Einsetzrichtung der Pfanne relativ zu der durch den Scheitelpunkt verlaufenden Symmetrieachse bestimmt, umso geringer ist — gleiche Kräfte vorausgesetzt — die eine Deformation der Laschen hervorrufende Kraftkomponente in Richtung dieser Symmetrieachse, umso geringer muss also der Verformungswiderstand der Laschen in dieser Richtung sein.

Soll die neue Pfanne gleichzeitig für das linke und das rechte Hüftgelenk eingesetzt werden, so ist es vorteilhaft, wenn die Laschen symmetrisch zu einer die Zapfenachse enthaltenden Mittelebene des Pfannenkörpers angeordnet sind, wobei der Winkel zwischen der Mittelebene und der Lasche am zweckmässigsten $\pi/3$ beträgt.

An- und Einwachsen von Gewebe an bzw. in die künstliche Hüftgelenkspfanne kann gefördert werden, wenn deren Aussenfläche, d.h. ihre Kontaktfläche zum Knochen, mit einem — vorzugsweise mehrlagigen — Metallnetz belegt ist. Wird die neue Pfanne aus Kunststoff gefertigt, so haben die Metallnetze den zusätzlichen Vorteil, dass sie eine Barriere zwischen Kunststoff und Knochen bzw. Körperflüssigkeit bilden, durch die der Kunststoff langzeitlich zersetzt werden kann.

Im folgenden wird die Erfindung anhand eines Ausführungsbeispiels im Zusammenhang mit der Zeichnung näher erläutert.

Fig. 1 ist ein Schnitt I-I von Fig. 2 durch die neue Hüftgelenkspfanne;

Fig. 2 gibt eine Aufsicht auf den Pfannenkörper in Richtung seiner Symmetrieachse wieder;

Fig. 3 stellt vergrössert den Schnitt III-III von Fig. 2 dar.

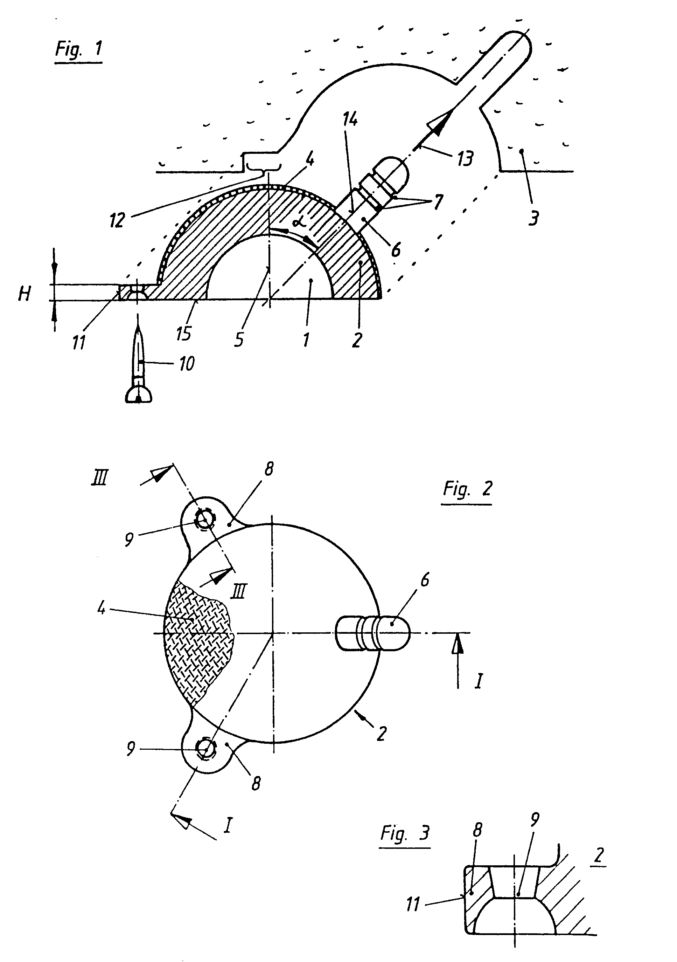

Die den nicht dargestellten Gelenkkopf einer Femurkopfprothese aufnehmende Pfannenschale 1 (Fig. 1) der Hüftgelenkspfanne ist in bekannter Weise in einen, mindestens nahezu halbkugelförmigen Pfannenkörper 2 eingearbeitet; dieser besteht vorzugsweise aus Kunststoff und ist auf seiner Aussenfläche, die die Kontaktfläche zum Knochengewebe darstellt, mit einem — vorzugsweise aus mehr als zwei Lagen bestehenden — Metallgitter 4 versehen. Dieses besteht aus einem der als besonders gewebefreundlich bekannten Metalle, wie Titan, Tantal, Niob, Zirkon oder Legierungen mit diesen Metallen als Basiswerkstoffen. Das Netz oder Gitter 4 bildet somit eine gewebefreundliche Oberflächenstruktur, in die im Laufe der Zeit Knochengewebe einwächst, wodurch der Pfannenkörper fest und knochenzementfrei im Knochen verankert wird.

Die Befestigung des Gitters 4 auf der Kontaktfläche des Pfannenkörpers 2, der vorzugsweise aus einer der in der Implantat-Technik bekannten und vielfach verwendeten Polyäthylen-Modifikationen hergestellt ist, erfolgt in einfacher Weise dadurch, dass das Gitter 4 in den durch erhöhte Temperatur erweichten Kunststoff durch erhöhten Druck eingepresst wird.

In einem Winkel $\alpha$ zu seiner Symmetrieachse 5 steht aus dem Pfannenkörper 2 ein Verankerungszapfen 6 hervor, der mit einer Rillenstruktur 7 für das Einwachsen von Gewebe versehen ist. Die Achse 14 des Zapfens 6 weist dabei in die Richtung (Pfeil 13), in der der Pfannenkörper 2 in das operativ entsprechend vorbereitete Becken 3 eingesetzt wird.

In einem Winkel von 120° zu einer Mittelebene durch die Zapfenachse 14 sind an dem an sich randlosen Pfannenkörper 2 zwei Laschen 8 vorgesehen, die eine Bohrung 9 aufweisen; die Bohrungen 9 sind äquatorseitig halbkubelförmig und knochenseitig trichterförmig oder konisch erweitert, wie besonders aus Fig. 3 zu erkennen ist. Diese Erweiterungen haben die Aufgabe, in gewissem Umfang die Richtung der Verankerungsschrauben 10 (Fig. 1) zu variieren, deren Köpfe deshalb an die äquatorseitige Hohlkugelform der Bohrungen 9 angepasst sind.

Die periphere Aussenform 11 der Laschen 8 ist erfindungsgemäss mindestens nahezu parallel zur Achse 5, d.h. sie kann unter Umständen auch in Richtung auf den Äquator 15 zur Achse 5 — aber nicht in die entgegengesetzte Richtung — leicht geneigt sein.

In Verbindung mit einer entsprechenden Ausfräsung 12 des Knochens 3 (Fig. 1) bewirkt die von der Einsetzrichtung abweichende periphere Aussenform 11, dass zwischen dem Zapfen 6 und den Laschen 8 ein Klemmsitz für eine Primärverankerung entsteht, ohne dass der Pfannenkörper 2 unzulässig verformt wird.

Zwischen der Höhe H, der Laschen 8 und dem Winkel α besteht dabei eine Abhängigkeit, die, wie bereits erwähnt, durch die Notwendigkeit einer gewissen Steife der Lasche 8 gegen ein Verbiegen durch in Richtung der Achse 5 wirkende Kräfte bedingt ist; da die Kraftkomponente in der genannten Achsrichtung umso grösser ist je kleiner der Winkel α für die Einsetzrichtung ist, ist die Höhe H umso grösser je kleiner α ist und umgekehrt.

## Patentansprüche

1. Randlose Hüftgelenkspfanne mit einem in die von der Symmetrieachse (5) der Pfanne (2) abweichende Einsetzrichtung (13) weisenden, aus der äusseren Oberfläche hervorstehenden Zapfen (6), dadurch gekennzeichnet, dass aus dem Umfang des Pfannenkörpers (2) in der Äquatorebene zwei Verankerungslaschen (8) mit Bohrungen (9) hervorragen, deren Achsen parallel zur Symmetrieachse (5) des Pfannenkörpers (2) verlaufen, und dass ferner die periphere Aussenform (11) der Laschen (8) ebenfalls mindestens nahezu parallel zur Symmetrieachse (5) des Pfannenkörpers (2) verläuft, so dass im eingebauten Zustand zwischen dem Zapfen (6) und den Laschen (8) ein Klemmsitz zur Primärfixierung der Pfanne entsteht.

2. Hüftgelenkspfanne nach Anspruch 1, dadurch gekennzeichnet, dass die Laschenhöhe (H) von dem Winkel α abhängt, den die Zapfenachse (14) mit der Symmetrieachse (5) des Pfannenkörpers (2) bildet, wobei die Laschhöhe (H) bei grossen Winkel α relativ klein ist und umgekehrt.

3. Hüftgelenkspfanne nach Anspruch 1, dadurch gekennzeichnet, dass die Laschen (8) symmetrisch zu einer die Zapfenachse (14) enthaltenden Mittelebene des Pfannenkörpers (2) angeordnet sind.

4. Hüftgelenkspfanne nach Anspruch 3, dadurch gekennzeichnet, dass der Winkel zwischen der Mittelebene und den Laschen (8) π/3 beträgt.

5. Hüftgelenkspfanne nach Anspruch 1, dadurch gekennzeichnet, dass die Bohrungen (9) zur Äquatorebene hin zu Hohlkugelflächen und etwa von der Mitte der Laschenhöhe (H) ab gegen den Knochen hin zu einem Konus erweitert sind.

6. Hüftgelenkspfanne nach Anspruch 1, dadurch gekennzeichnet, dass die Aussenfläche des Pfannenkörpers (2) mit einem Metallnetz (4) belegt ist.

## Claims

1. An edgeless acetabulum having a pin (6) which extends in the insertion direction (13), the same deviating from the axis (5) of symmetry of the acetabulum (2), and which projects from the outside surface, characterised in that two anchoring tabs (8) extend from the periphery of the acetabulum socket (2) in the equatorial plane and are formed with bores (9) whose axes extend parallel to the axis (5) of symmetry of the socket (2), and the external peripheral shape (11) of the tabs (8) also extends at least substantially parallel to the axis (5) of symmetry of the socket (2), so that a clamping fit for primary fixing of the socket arises in the fitted state between the pin (6) and the tabs (8).

2. An acetabulum according to claim 1, characterised in that the tab height (H) depends on the angle α which the pin axis (14) forms with the axis (5) of symmetry of the socket (2), the tab height (H) being relatively small for large angles α and vice versa.

3. An acetabulum according to claim 1, characterised in that the tabs (8) are disposed symmetrically of a centre-plane of the socket (2), such plane containing the pin axis (14).

4. An acetabulum according to claim 3, characterised in that the angle between the centre-plane and the tabs (8) is π/3.

5. An acetabulum according to claim 1, characterised in that the bores widen towards the equatorial plane into hollow spherical surfaces and away from the centre of tab height (H) towards the bone into a cone.

6. An acetabulum according to claim 1, characterised in that the outside surface of the socket (2) is covered by a metal mesh (4).

## Revendications

1. Calotte d'articulation coxofémorale, dépourvue de rebord et présentant un tenon (6) qui dépasse au-delà de la surface extérieure et est orienté dans la direction d'insertion (13) déviant de l'axe de symétrie (5) de la calotte (2), caractérisée par le fait que deux pattes d'ancrage (8), saillant dans le plan équatorial à partir du pourtour du corps (2) de la calotte, comportent des perçages (9) dont les axes s'étendent parallèlement à l'axe de symétrie (5) du corps de calotte (2); et par le fait que, en outre, la configuration extérieure périphérique (11) des pattes (8) s'étend, elle aussi, au moins à peu près parallèlement à l'axe de symétrie (5) du corps de calotte (2), ce qui confère en condition incorporée, entre le tenon (6) et les pattes (8), une assise par coincement en vue de la fixation primaire de la calotte.

2. Calotte d'articulation coxofémorale selon la revendication 1, caractérisée par le fait que la hauteur (H) des pattes dépend de l'angle α que l'axe (14) du tenon forme avec l'axe de symétrie (5) du corps de calotte (2), la hauteur (H) des pattes étant relativement petite en présence de grands angles α, et inversement.

3. Calotte d'articulation coxofémorale selon la revendication 1, caractérisée par le fait que les pattes (8) sont agencées symétriquement par rapport à un plan médian du corps de calotte (2), par lequel passe l'axe (14) du tenon.

4. Calotte d'articulation coxofémorale selon la revendication 3, caractérisée par le fait que l'angle compris entre le plan médian et les pattes (8) mesure $\pi/3$.

5. Calotte d'articulation coxofémorale selon la revendication 1, caractérisée par le fait que les perçages (9) sont évasés vers le plan équatorial, pour former des surfaces sphériques creuses, puis en direction de l'os, sensiblement à partir du centré de la hauteur (H) des pattes, pour former un cône.

6. Calotte d'articulation coxofémorale selon la revendication 1, caractérisée par le fait que la surface extérieure du corps de calotte (2) est revêtue d'une treillis métallique (4).

Fig. 1

Fig. 2

Fig. 3